# EUROPEAN PATENT SPECIFICATION

(11) **EP 3 473 941 B1**
(45) Date of publication and mention of the grant of the patent: **18.05.2022**
(21) Application number: 18200474.7
(22) Date of filing: 15.10.2018
(51) Int. Cl.: F24F 8/10, F24F 3/16, F24F 13/28, F24F 7/06, F24F 13/20, B01J 23/50, B01J 35/00, F24F 8/22

(54) **AMBIENT AIR PURIFICATION UNIT**
UMGEBUNGSLUFTREINIGUNGSEINHEIT
UNITÉ DE PURIFICATION DE L'AIR AMBIANT

(30) Priority: 18.10.2017 IT 201700117827
(43) Date of publication of application: 24.04.2019
(73) Proprietor: Sagula, Maria, 24040 Verdellino (BG) (IT)
(72) Inventor: SAGULA, Maria, 24040 VERDELLINO (BG) (IT); SANSO', Giovanni, 20125 Milano (IT)
(74) Representative: Vanosi, Adelio Valeriano

(56) References cited:
- WO-A1-2005/044446
- DE-B3-102006 049 815
- US-A- 5 755 867
- US-A1- 2005 129 591
- US-B2- 8 328 917

## Description

### FIELD OF THE INVENTION

The present invention relates to an ambient air purification unit.

In particular, the invention refers to a purification unit that can be integrated into ventilation systems in new or existing buildings and can be used for the purification of entire buildings or solely certain rooms thereof. The buildings or rooms within which the unit finds application are those where it is useful to be able to monitor, including therein in biological terms, the ventilation air, for example healthcare facilities, hospitals, waiting rooms for medical and outpatient surgeries, dental surgeries, pharmaceutical, dietary, and cosmetics manufacturing facilities, public environments, large-scale kitchens, community meeting rooms, restaurants, school canteens, meeting rooms, cinemas, buses, and means of public transport etc. Essentially, any public or private places where there may be a risk of spreading infections or where one wishes to prevent the spread of infections.

### BACKGROUND ART

Commonly known ventilation systems for constructions such as rooms or entire buildings, include ducts equipped with air intake vents located inside the construction; the intake air is filtered roughly and may be heated or cooled, and may be supplemented with air taken from the outside and subsequently reintroduced into the construction by means of suitable vents or diffusers.

The patent literature US 8,328,917 B2 provides the features of the preamble of claim 1 and discloses a system and a method for photocatalytic oxidation and air filtration using a substrate with photocatalyst particles powder coated thereon.

The commonly known systems are unable to appropriately sanitise and remove odours from the air present in the construction.

### SUMMARY OF THE INVENTION

The object of the present invention is to provide an ambient air purification unit which is more effective in terms of the filtration and purification of the air present in the closed constructions than currently known air purification units.

Another object of the invention is to provide a unit that is simple and easy to install, manage, and service.

This and other objects are achieved by means of an air filtration unit produced according to the technical teachings of the claims annexed hereto.

### BRIEF DESCRIPTION OF THE FIGURES

Further characteristics and advantages of the invention will become clearer in the description of a preferred but not exclusive embodiment of the device, illustrated - by way of a non-limiting example - in the drawings annexed hereto, in which:
Figure 1 shows a perspective view of a unit according to the present invention, in which one wall of the frame has been removed to render the internal components visible;
Figure 2 is an enlarged, simplified, partial view of a filter in the filtration unit;

### DETAILED DESCRIPTION OF THE INVENTION

With reference to the figures stated, reference number 1 is used to denote, as a whole, an air purification unit.

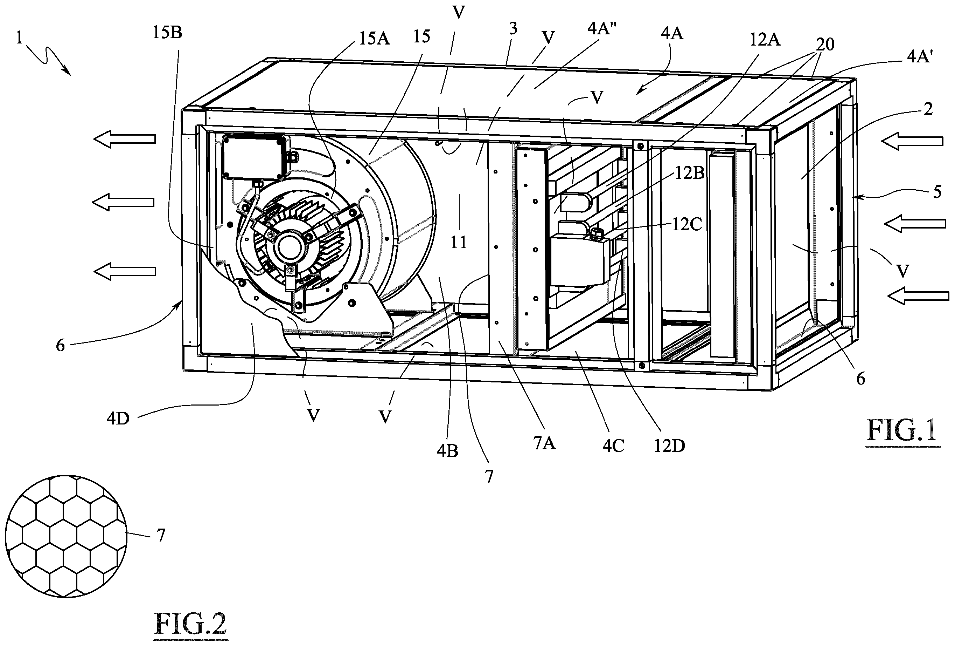

As can be seen in Figure 1, the air purification unit 1, comprises a frame 3 supporting at least four boundary walls 4A, 4B, 4C, 4D, some or all of which can be removed, preferably fastened to the frame with screws.

The walls may be made of continuous metal sheet or cropped metal sheets, as in the case of the upper wall (in the figure) denoted 4A, which is formed of a first part 4A', which is smaller, and a larger part 4A".

The frame and the walls, together, define a box-shaped construction which has one end open, to form an area of suction 5, and another end, opposite the first one, in which there is a delivery flow 6 from the unit 1.

It must be said that in the embodiment illustrated, the delivery flows inwards longitudinally with respect to the frame, but obviously the said delivery could also flow inwards in an angled or transverse direction.

The area of suction 5 comprises, or rather is associated with, a first filter 2 for separation of the rough and fine dust, which can also, advantageously, isolate any emulsions present in the suctioned air.

The first filter 2 may be made of polyester, paper, or metal, and may be - advantageously - of the pleated filter cell type.

At the base thereof, the filter may feature a particulate collecting tank.

The first filter may be a box filter, so that the said filter can be replaced if necessary, for example by removing the panel 4A' of the wall 4A.

After the first filter 2 (in the direction in which the air is flowing inside unit 1), a second filter 7 is present.

The second filter 7 is endowed with macroscopic openings, which are advantageously configured in a honeycomb arrangement, as shown in Figure 2. The area of passage of the openings may be between 3.00 mm² and 3.36 mm².

The second filter 7 comprises titanium dioxides and also, optionally, silver ions (soluble silver salts such as nitrate), which are integrated into the constituent material of the filter and/or applied thereto by means of a paint.

The second filter may also be removable for the cleaning or replacement thereof. The said filter is housed in a dedicated frame 7A to which a plurality of UV-C lamps 12A, 12B, 12C, 12D, are fixed, which are preferably four in number although a single lamp would suffice. In the present description, reference number 12 will denote the lamps all together.

For operation of the unit, at least one fan is featured, downstream of the second filter 7, one of whose outlets flows into the delivery 6 of the unit 1.

The fan 15, which is advantageously of the centrifugal type, and preferably in compliance with ErP energy efficiency regulations, has an intake 15A fluidly associated with the duct downstream of the second filter 7.

As mentioned earlier, inside the duct, upstream of the fan 15, there is at least one UV-C lamp 12. The at least one UV-C lamp (shortwave ultraviolet lamp) is positioned so as to illuminate an area upstream and downstream of the second filter 7 through the macroscopic openings in the second filter 7.

Indeed, the interior of the boundary walls 4, or at least the part facing the inside of the duct, is coated with a paint V comprising titanium dioxide and, optionally, silver nitrate (before being applied to the walls).

For example, before being applied to the panels (with a brush or a spray), the paint V comprises dispersed titanium dioxide anatase in between 0.5 and 3% by weight prior to application, and/or comprises nitrate silver ranging from 0.1 to 1% in weight prior to application.

Advantageously, the paint V is also applied to the first and the second filter, to increase the power thereof to sanitise the unit 1.

To conclude the description, it must be specified that downstream of the second filter 7, a pressure switch (11) may be featured for detecting a degree of clogging of one or both the first filter 2 and second filter 7.

Furthermore and advantageously, the fan 15 and the pressure switch 11 may be associated with a control unit, optionally also associated with the at least one UV-C lamp 12.

The operation of the invention is clear to a person skilled in the art and is essentially as follows.

Titanium dioxide, advantageously present in the paint as nanoparticles, has an antismog, antibacterial effect, and develops a hydrophilic effect on the surfaces and therefore a self-cleaning set-up; furthermore, silver nitrate, optionally present in the paint V, provides a further antibacterial effect, even in the absence of light, and acts as a catalyst.

Unit 1 is therefore suitable for purifying air to remove suspended solid microparticulate, bacteria, viruses, emulsions, fumes and noxious odorous substances.

The unit can be advantageously integrated into a system for the aeration of a building or of only certain rooms thereof. The unit can also be used as a standalone device for the purification of specific areas or rooms.

Indeed, the series of filters soaked or otherwise coated with paint comprising titanium dioxide nanoparticles, activated by silver nitrate nanoparticles and by UV-C lamps (whose emission peaks at preferably 253.7 nm) with an appropriate output, together with the use of the said paint V on many or all the surfaces inside the unit, provides a valid and effective purification of all the air handled by the unit.

Advantageously, the interior of the fan may also be painted with the aforesaid paint V.

It should be noted that the drop in air flow which occurs during the passage through the filters and the UV-C lamp section leads to a reduction in air pressure and consequently air temperature.

As the intake air is damp, there may be a partial condensation of the moisture, which - together with the lipophilicity of the titanium dioxide- plays a useful role in keeping catalytic activity of the unit high at all times; any non-solid noxious components which are not retained by the filters are therefore eliminated through oxidation.

Essentially, the photocatalytic reaction generates radicals which, through complex reactions, oxidise most of the organic pollutants to CO₂, i.e. to complete removal of the organic contaminant from the fluid.

Various embodiments of the innovation have been disclosed herein, but further embodiments may also be conceived without departing from the scope of the appended claims.

## Claims

1. An air purification unit (1), comprising a support frame (3) of at least four boundary walls (4A, 4B, 4C, 4D), at least one of which is removable, the frame and walls defining a box-like structure defining a suction (5) and a delivery (6) of the unit respectively placed in opposite positions of the box-like structure, the suction (5) comprising a first separating filter of coarse and fine dust, the first filter (2) being followed, in a flow direction of the air within the unit, by at least one UV-C lamp (12) followed by a second filter (7) having macroscopic openings, wherein
downstream of the second filter (7) being provided at least one fan whose outlet flows in the delivery (6) of the unit (1), and an intake of which is fluidly associated to the inside the duct, downstream of the second filter (7), **characterised in that**
- at least the boundary walls (4) facing the inside of the duct, being coated with a paint (V) comprising titanium dioxide;
- the at least one UV-C lamp (12) being positioned so as to illuminate, an area placed upstream of said second filter (7) and also an area placed downstream of said second filter (7), through the macroscopic openings of the second filter (7).

2. The unit according to the preceding claim, wherein the first filter (2) is provided, at its base, with a particulate collecting tank

3. The unit according to the preceding claim, wherein at least one UV-C lamp is fixed to a structure supporting the second filter (7).

4. The unit according to claim 1, wherein the openings of the second filter (7) are honeycomb-shaped.

5. The unit according to one or more of the preceding claims, wherein downstream of the second filter (7) a pressure switch (11) is provided for detecting a degree of clogging of one or both the first (2) and second filter (7).

6. The unit according to one or more of the preceding claims, wherein the fan (15) and the pressure switch (11) are associated with a control unit, optionally also associated with the UV-C lamp.

7. The unit according to one or more of the preceding claims, wherein the first (2) and / or the second filter (7) are removable for maintenance or replacement, and / or wherein the first filter (2) is made of polyester, or paper or metal and / or is of the type with pleated filter cells.

8. The unit according to one or more of the preceding claims, wherein the second filter (7) and / or the first filter comprises titanium dioxide, and optionally also silver ions, and / or wherein the first (2) and / or the second filter (7) is impregnated or varnished with a paint (V) comprising, prior to application, titanium dioxide and optionally silver nitrate.

9. The unit according to one or more of the preceding claims, wherein the paint (V) comprises dispersed titanium dioxide anatase between 0.5 and 3% by weight prior to application, and / or comprising nitrate silver ranging from 0.1 to 1% in weight prior to application.

## Patentansprüche

1. Luftreinigungseinheit (1), einen Stützrahmen (3) mit mindestens vier Begrenzungswänden (4A, 4B, 4C, 4D) umfassend, von denen mindestens eine entfernbar ist, wobei der Rahmen und die Wände eine kastenartige Struktur definieren, die eine Ansaugung (5) und eine Abgabe (6) der Einheit definiert, die jeweils an gegenüberliegenden Positionen der kastenartigen Struktur angeordnet sind, wobei die Ansaugung (5) einen ersten Trennfilter für Grob- und Feinstaub umfasst, wobei dem ersten Filter (2) in einer Strömungsrichtung der Luft innerhalb der Einheit mindestens eine UV-C-Lampe (12) folgt, gefolgt von einem zweiten Filter (7) mit makroskopischen Öffnungen, wobei
stromabwärts des zweiten Filters (7) mindestens ein Lüfter bereitgestellt ist, dessen Auslass in die Abgabe (6) der Einheit (1) strömt und dessen Einlass in Fluidverbindung mit dem Inneren des Kanals, stromabwärts des zweiten Filters (7), steht, **dadurch gekennzeichnet, dass**
- mindestens die dem Inneren des Kanals zugewandten Begrenzungswände (4) mit einer Farbe (V), die Titandioxid umfasst, beschichtet sind;
- die mindestens eine UV-C-Lampe (12) so positioniert ist, dass sie einen Bereich, der stromaufwärts des zweiten Filters (7) angeordnet ist, und auch einen Bereich, der stromabwärts des zweiten Filters (7) angeordnet ist, durch die makroskopischen Öffnungen des zweiten Filters (7) beleuchtet.

2. Einheit nach dem vorhergehenden Anspruch, wobei der erste Filter (2) an seiner Basis mit einem Partikelsammelbehälter versehen ist.

3. Einheit nach dem vorhergehenden Anspruch, wobei mindestens eine UV-C-Lampe an einer Struktur befestigt ist, die den zweiten Filter (7) trägt.

4. Einheit nach Anspruch 1, wobei die Öffnungen des zweiten Filters (7) wabenförmig sind.

5. Einheit nach einem oder mehreren der vorhergehenden Ansprüche, wobei stromabwärts des zweiten Filters (7) ein Druckschalter (11) zum Erkennen eines Verstopfungsgrades eines oder beider von dem ersten (2) und dem zweiten Filter (7) bereitgestellt ist.

6. Einheit nach einem oder mehreren der vorhergehenden Ansprüche, wobei der Lüfter (15) und der Druckschalter (11) einer Steuereinheit zugeordnet sind, optional auch der UV-C-Lampe zugeordnet ist.

7. Einheit nach einem oder mehreren der vorhergehenden Ansprüche, wobei der erste (2) und/oder der zweite Filter (7) zur Wartung oder zum Austausch entfernbar sind, und/oder wobei der erste Filter (2) aus Polyester oder Papier oder Metall hergestellt ist und/oder vom Typ mit gefalteten Filterzellen ist.

8. Einheit nach einem oder mehreren der vorhergehenden Ansprüche, wobei der zweite Filter (7) und/oder der erste Filter Titandioxid und optional auch Silberionen umfasst, und/oder wobei der erste (2) und/oder der zweite Filter (7) mit einer Farbe (V) getränkt oder lackiert ist, die vor dem Auftragen Titandioxid und optional Silbernitrat umfasst.

9. Einheit nach einem oder mehreren der vorhergehenden Ansprüche, wobei die Farbe (V) vor dem Auftragen dispergiertes Titandioxid-Anatas zwischen 0,5 und 3 Gew.-% umfasst und/oder vor dem Auftragen Nitratsilber im Bereich von 0,1 bis 1 Gew.-% umfasst.

## Revendications

1. Unité de purification d'air (1), comprenant un cadre support (3) d'au moins quatre parois de délimitation (4A, 4B, 4C, 4D), dont au moins l'une est amovible, le cadre et les parois définissant une structure en forme de caisson définissant une aspiration (5) et un refoulement (6) de l'unité respectivement placés à des positions opposées de la structure en forme de caisson, l'aspiration (5) comprenant un premier filtre de séparation des poussières grossières et fines, le premier filtre (2) étant suivi, dans une direction d'écoulement de l'air à l'intérieur de l'unité, par au moins une lampe UV-C (12) suivie d'un second filtre (7) ayant des ouvertures macroscopiques, dans laquelle
en aval du second filtre (7) est prévu au moins un ventilateur dont l'évacuation s'écoule dans le refoulement (6) de l'unité (1), et dont une admission est fluidiquement associée à l'intérieur du conduit, en aval du second filtre (7), **caractérisé en ce que**
- au moins les parois de délimitation (4) tournées vers l'intérieur du conduit, sont revêtues d'une peinture (V) comprenant du dioxyde de titane ;
- l'au moins une lampe UV-C (12) est positionnée de manière à éclairer une zone placée en amont dudit second filtre (7) et également une zone placée en aval dudit second filtre (7), à travers les ouvertures macroscopiques du second filtre (7).

2. Unité selon la revendication précédente, dans laquelle le premier filtre (2) est pourvu, à sa base, d'un réservoir collecteur de particules.

3. Unité selon la revendication précédente, dans laquelle au moins une lampe UV-C est fixée à une structure supportant le second filtre (7).

4. Unité selon la revendication 1, dans laquelle les ouvertures du second filtre (7) sont en forme de nid d'abeilles.

5. Unité selon une ou plusieurs des revendications précédentes, dans laquelle en aval du second filtre (7) un pressostat (11) est prévu pour détecter un degré de colmatage d'un ou des deux des premier (2) et second filtres (7).

6. Unité selon une ou plusieurs des revendications précédentes, dans laquelle le ventilateur (15) et le pressostat (11) sont associés à une unité de commande, éventuellement également associés à la lampe UV-C.

7. Unité selon une ou plusieurs des revendications précédentes, dans laquelle le premier (2) et/ou le second filtres (7) sont amovibles pour l'entretien ou le remplacement, et/ou dans laquelle le premier filtre (2) est en polyester, ou en papier ou en métal et/ou est du type à cellules filtrantes plissées.

8. Unité selon une ou plusieurs des revendications précédentes, dans laquelle le second filtre (7) et/ou le premier filtre comprend du dioxyde de titane, et éventuellement également des ions d'argent, et/ou dans laquelle le premier (2) et/ou le second filtres (7) est imprégné ou verni d'une peinture (V) comprenant, avant application, du dioxyde de titane et éventuellement du nitrate d'argent.

9. Unité selon une ou plusieurs des revendications précédentes, dans laquelle la peinture (V) comprend du dioxyde de titane anatase dispersé entre 0,5 et 3 % en poids avant application, et/ou comprend du nitrate d'argent allant de 0,1 à 1 % en poids avant application.
